(19)

Europäisches
Patentamt
European
Patent Office
Office européen
des brevets

(11) **EP 1 262 764 B1**

(12) **EUROPEAN PATENT SPECIFICATION**

(45) Date of publication and mention
of the grant of the patent:
**11.04.2007 Bulletin 2007/15**

(51) Int Cl.:
*G01N 21/77* (2006.01)    *G01N 33/50* (2006.01)
*G01N 21/64* (2006.01)    *B01L 3/00* (2006.01)

(21) Application number: **01401376.7**

(22) Date of filing: **25.05.2001**

(54) **Method for the detection of reactions and metabolic changes with temperature-sensitive fluorescent material**

Verfahren zur Bestimmung der Reaktionen und der Metabolischen Aktivitat mit Fluoreszentem temperaturempfindlichem Material

Procédé pour la détermination des réactions et de l'activité métabolique avec materiau fluorescent thermosensible

(84) Designated Contracting States:
**DE FR GB IT NL**

(43) Date of publication of application:
**04.12.2002 Bulletin 2002/49**

(73) Proprietor: **Corning Incorporated**
**Corning, NY 14831 (US)**

(72) Inventors:
• **Lemee, Valerie J. C.**
**SP FR 02-12,**
**Corning, NY 14831 (US)**
• **Pecheul,Marylene D. M.**
**SP FR 02-12,**
**Corning, NY 14831 (US)**

• **Marque, Pascal**
**SP FR 02-12,**
**Corning, NY 14831 (US)**
• **Root, David M.**
**SP FR 02-12,**
**Corning, NY 14831 (US)**

(74) Representative: **Marchant, James Ian et al**
**Elkington and Fife LLP,**
**Prospect House,**
**8 Pembroke Road**
**Sevenoaks,**
**Kent TN13 1XR (GB)**

(56) References cited:
**WO-A-99/35496**     **GB-A- 2 333 153**
**US-A- 4 838 665**     **US-A- 5 738 825**
**US-A- 6 045 259**     **US-A- 6 132 958**

EP 1 262 764 B1

**Description**

**FIELD OF THE INVENTION**

This invention relates to assaying or screening substances by detection of chemical reactions and metabolic changes in biological materials using a light signal. More particularly, the present invention relates to methods of detecting reactions involving chemicals, biomolecules and other compounds and metabolic changes in cells, the methods utilizing the measurement of a light signal. According to one embodiment the light signal is generated by a fluorescent material, the fluorescence of which depends on temperature.

**BACKGROUND OF THE INVENTION**

The drug discovery process is a multiple step process involving identification of disease targets, assay development and validation, high throughput primary screening of compound libraries, hit validation in secondary screens, lead optimization, Absorption Distribution Metabolism and Excretion (ADME) and toxicity in pre-clinical trials. This long process eventually leads to a drug candidate that enters clinical trial phases. The assay development and validation phase is used to optimize the labeling system and detection method to be used for a robust, low background and low variability screen. Standard labels are either radioactive elements or fluorescent/luminescent or absorbing compounds. High throughput screening of compound libraries requires automated parallel handling and processing of labeled biological reagents and compound mixtures. Standard screens are performed using microarray chips, microfluidic chips, and microtiter plates (hereinafter "microplates") with 96, 384 or 1536 wells compatible with fluid handling equipment and detection instruments. Recent developments of Charge Coupled Device (CCD) based detection instruments and compound arraying techniques allow for screening compounds in formats having higher densities than standard microplates.

Cell-based assays are often used in the drug discovery process. They are particularly useful when the drug target is a transmembrane receptor or an ion-channel. Scintillation proximity assays, as well as fluorescence assays have been designed to monitor the physiological state of the cells, for example, by monitoring the level of second messenger concentrations (e.g., cAMP, $Na^+$, DAG, etc.). In some cases, a cell lysis step is required for the measurement. In other cases, the cells are transfected with a fluorescent protein, the fluorescent properties of which depend on the concentration of a second messenger. High content screens also exist in which labeled molecules are used to visualize receptor endocytosis, recycling and intracellular trafficking of messenger biomolecules, providing additional information on the physiological effects cause by receptor/ligand binding.

It is advantageous to monitor the cell physiological state without using time consuming and labor intensive labeling steps such as genetic engineering or even perfusion. An effective way of performing such assays is by measuring acidification rates of the medium in which cells are suspended (J.C. Owicki, J. Wallace Parce, 'Biosensors Based on the Energy Metabolism of Living Cells: The Physical Chemistry and Cell Biology of Extracellular Acidification, Biosensors & Bioelectronics, 1992, 7, 255-272). Another way of performing such assays involves using a microphysiometer that uses highly sensitive pH sensors, which is available from Molecular Devices Corp., Sunnyvale, CA. Still another way of monitoring the physiological state of cultured cells is by measuring the heat flow using a calorimetric technique. However, none of these label-free technologies is compatible with the requirement of high throughput for screening thousands of compounds from large chemical libraries.

Imaging infrared thermography is another technology that is used to monitor physiological and molecular events that elicit a thermogenic response in animals, plants, tissues, cells and cell-free systems. This method can be used for screening drug candidates. Whereas this method can provide throughput, the detection principle is difficult to master because it cannot easily produce absolute temperature measurements. Moreover, besides the performance of the detector used for imaging infrared thermography, temperature sensitivity is limited by the overall system noise. Temperature sensitivity also depends on the materials used and on the emissive and reflective properties of the last interface between the imaged object and air in front of the detector.

There are also numerous methods of optically monitoring biological interactions and/or chemical reactions based on the measurement of refractive index. Besides standard refractive index-based methods, such methods include evanescent wave-based methods using for example, surface plasmon resonance or optical resonant structures such as grating couplers and resonant mirrors. For example, United States Patent Number 5,738,825 describes an optical biosensor including a detection cell including a transparent base plate and a sample plate on the base plate. The sample plate has a matrix of wells to receive a sample, and the base plate includes a diffraction grating and a waveguiding film to incouple incident light into the waveguiding film adjacent the bottom of the well structure. The incoupled light field generates a diffracted light field to enable detection of a change in the effective refractive index of the waveguiding film.

In the optical detection methods described above, the biological interactions monitored are performed in a liquid medium, typically an aqueous medium, in contact with the sensing area. One limitation of these optical detection methods is that the refractive index of the sensing structure may vary with temperature.

Another limitation is that there may be refractive index variations among various locations of the sensing structure. For example, when the sensing structure is a microplate containing a plurality of wells (e.g., 6, 24, 96 or 384 wells), there can be temperature variations in different wells of the same microplate. This temperature variation in different wells may be 3 °C or higher. In addition, for systems that monitor temperature in a liquid medium, the variation of refractive index related to the biological event is further masked by the refractive index variations of the liquid medium itself. Different volumes of liquids in different wells affect the path length of the optical signal, leading to further signal variations in optical detection systems. This difference in volume can be due to the fact that a different volume of liquid has been dispensed in an individual well, or due to evaporation of the liquid from the wells. These well-to-well volume differences either generate signal variations or must be compensated by some other means. One commercially available detector available from Molecular Devices, Sunnyvale, California corrects for well volume differences. However, it would be advantageous and less complicated if a system did not require corrections in the volume of liquid in each well to simplify the analysis of samples in microplates. Furthermore, there is no known method or apparatus that corrects for refractive index changes due to temperature changes in the local sensing area.

Biological interactions monitored by the methods referenced above typically translate in refractive index variations ranging from $10^{-2}$ down to $10^{-5}$ and lower. Therefore, the measurements must be performed in an environment in which temperature is rigorously controlled, preferably with temperature variations lower than 0.1°C to preserve accuracy and sensitivity of the measurement.

GB-A-2 333 153 relates to measuring the temperature of a cell by using a temperature-sensitive fluorophone using a metabolic probe which comprises a solid substrate containing the temperature-sensitive fluorophone embedded in a polymer. When the fluorophone is excited with the appropriate light, the fluorophone emits a detectable fluorescent signal and when a living cell is placed on the metabolic probe the intensity of the fluorescent signal is effected by the metabolic rate of the cell. The method is applicable for example to the detection of tumor cells.

US-A-6,132,958 relates to a fluorescent bead that can be used for determining the temperature and/or metabolic state of a single cell contained in a cell/tissue sample, more particularly in the diagnosis of abnormal metabolism of a cell. The bead may comprise at least one temperature-sensitive fluorophone and at least one temperature-insensitive fluorophone embedded in a polymeric material.

WO-A-99/35496 relates to the screening of test compounds for bioactivity by contacting an array of test compounds with a detector layer comprising physiologically viable cells for example in the form of a monolayer. A cell response is indicative of bioactivity and for example the detection step may involve a change in a fluorescence or luminescence property of the cell.

US-A-6,045,259 relates to a fiber-optic temperature sensor comprising a continuous crystalline fiber-optic high temperature sensor probe having a crystalline optical waveguiding region and a crystalline fluorescent temperature sensing tip at one end thereof. The fluorescent temperature sensing tip contains fluorescent ions that can be excited to fluoresce and produce a fluorescence emission.

There is a need to provide devices methods and systems capable of performing fast and reliable high throughput screening of cells and biomolecules. It would be desirable to perform the high throughput screening using standard instrumentation and a relatively simple method which would facilitate screening drug candidates for their interaction with another compound. In addition, for devices, methods and systems that utilize changes in refractive index to monitor metabolic changes in cells and interactions between and among biomolecules, it would be useful to provide the capability to monitor for and compensate for temperature-induced changes in refractive index.

## SUMMARY OF THE INVENTION

According to one aspect the present invention provides a method for assaying or screening substances proximate a sensing area of a surface comprising: detecting a light signal generated proximate the sensing area; measuring the temperature proximate the sensing area; measuring the refractive index of the sensing area; and determining the change in refractive index of the sensing area due to the change in temperature and adjusting the light signal in accordance with the change in refractive index.

Accordingly, the present invention generally provides methods for assaying samples, particularly samples including biomolecules or cells. These involve a sensing area which may include a substrate, for example, a microplate for assaying samples including a frame forming sidewalls of at least one well and a bottom portion that forms a bottom of at least one well. The bottom portion may include a fluorescent material in thermal communication with the at least one well. In a preferred aspect, the fluorescence of the fluorescent material changes as the temperature of the at least one well changes. Desirably, the fluorescent material is operative to produce a change in fluorescence to detect a chemical reaction, a biomolecular reaction, or a metabolic response of a cell.

A chemical reaction could simply involve detecting whether two different reactants produce an endothermic or exothermic reaction, such as the mixture of potassium hydroxide and water. An example of a biomolecular reaction is the binding of a biomolecule to another compound. Such binding typically results in a metabolic change in a biomolecule, which produces either a positive or negative heat of reaction. A metabolic response of a cell may be produced, for example, when a cell or cell fragment is contacted with a serum, which can be detected by monitoring the temperature of the cell to determine if there is a change in temperature. By monitoring the fluorescence of a fluorescent material having a temperature dependent fluorescence, the presence or absence of a reaction or metabolic change can be detected on a sample substrate such as a microarray of biomolecules or cells or a microplate well that incorporates such a fluorescent material.

The fluorescent material may be in the form of a film forming a layer adjacent the bottom portion of the substrate. Preferably the film has a thickness less than about 50 microns. Alternatively the substrate is a microplate, the bottom portion of the microplate has a bottom surface, and the layer is adjacent the bottom surface preferably the surface contacting a fluid contained in the well. In another alternative, the fluorescent material is embedded in the bottom portion of the substrate. The fluorescent material may include a rare-earth chelate. A particularly preferred rare earth chelate is EuTTA. Other preferred fluorescent materials include Rhodamine B, Erythrosin B or terthiophene. Other potential fluorescent materials that may be used including, but are not limited to EuFOD, EuTFC, TbFOD, EuBA, EuTHD, EuHFC, EuDBM, EuTA, EuTFA, EuDCM, TTED, TbTTA, TbTFA, TbBA, TbTHD, TbAA, and combinations thereof.

The substrate may include a biomolecule, a cell or a cell fragment bound to the surface of the substrate and a fluorescent material in thermal communication with a surface of the substrate. The fluorescent material has a temperature dependent fluorescence. Preferably, the substrate includes a microarray of biomolecules, cells or cell fragments on a surface thereof.

The invention may involve a method of detecting a chemical reaction, a biomolecular reaction or a metabolic change in a cell. The method includes providing a reaction substrate such as a microplate or a microarray chip including a fluorescent material. The fluorescence of the fluorescent material changes as the temperature of the fluorescent material changes. The method further includes placing a chemical, a biomolecule or a cell in reactive contact in or on the reaction substrate and monitoring the fluorescence of the fluorescent material.

As noted above, when two chemicals react, or when a biomolecule and a second compound react, such as when a target molecule and a receptor bind or when there is a metabolic change in a cell, a positive or negative thermal energy is created. This thermal energy change can be detected by monitoring fluorescence of a material having a fluorescence that changes with changing temperature. Thus, the method may include correlating a fluorescence reading with chemical or biomolecular reaction or a metabolic change in a cell by, for example, determining the change in temperature based on the change in fluorescence.

The method may relate to screening biomolecular or cellular assays. This method involves providing biomolecules or cells in an array of locations, such as in a microplate or a microarray chip used for high throughput screening of biomolecules or cells. The method further involves placing a compound in reactive contact with the biomolecules or cells in at least one of the locations and detecting the temperature change in the at least of one of the locations by detecting the change in fluorescence of at least one of the locations. Preferably, at least one of the array of locations contains a fluorescent material, the fluorescence of which changes with temperature.

The invention may also involve a system for high throughput screening of biomolecules or cells, The system includes a sample holder including an array of locations, the sample holder including a fluorescent material, the fluorescence of which changes with temperature. The system further involves providing a structure, method or device for contacting the biomolecules or cells with a compound in at least one of the array of locations. The system also includes a measurement device, for example, a fluorescence microthermal imaging device, for detecting the change in fluorescence of the fluorescent material as the temperature in at least one of the locations changes.

The invention may also involve an optical sensing system including a substrate in contact with a fluid containing a biomolecule or a cell, a waveguide associated with the substrate, a light source a light detector and a fluorescent material the fluorescence of which changes with changing temperature. According to this aspect of the invention, the waveguide may include one or more planar waveguides, optical fibers, grating structures, or combinations thereof. The optical sensing system may further include a processor for determining temperature changes in accordance with the changing in fluorescence of the fluorescent material. The processor is operative to receive an optical signal and correlate changes in temperature with changes in the refractive index of the substrate and/or the fluid. The processor is thus operative to adjust the optical signal in accordance with the correlated change in refractive index of the fluid and/or the substrate to provide a compensated optical signal for the temperature dependent refractive index change of the substrate or the fluid.

The method may also involve analyzing substances proximate a sensing area of a surface. The method includes the steps of detecting a light signal generated proximate the sensing area, measuring the temperature proximate the sensing area, measuring the refractive index of the sensing area, and determining the change in refractive index of the sensing area due to the change in temperature and adjusting the light signal in accordance with the change in refractive index. The sensing area may include a substrate and a fluid containing a cell or a biomolecule in contact with the substrate. A suitable substrate may include a microplate, a microarray chip or a microfluidic chip. Preferably, a fluorescent material having a temperature-dependent fluorescence is proximate the sensing area.

The invention provides a relatively simple and flexible method using standard instrumentation to detect chemical reactions, biomolecular reactions and metabolic changes in a cell, which facilitates high throughput screening of biomolecules. Additional advantages of the invention will be set forth in the following detailed description. It is to be understood that both the foregoing general description and the following detailed description are exemplary and are intended to provide further explanation of the invention as claimed.

**BRIEF DESCRIPTION OF THE DRAWINGS**

FIGS. 1A-1C show microplate structures having a fluorescent material associated with the microplates according to the invention;

FIGS. 2A-2B show schematic representations of systems for monitoring the change in temperature in a sample holder having an array of locations containing biomolecules;

FIG. 2C shows a schematic representation of a system for monitoring the change in temperature in a sample holder having an array of locations containing biomolecules and correlating the changes in temperature with refractive index change;

FIG. 3 is graph showing the absorption and emission spectra of a EuTTA/PMMA film on a microplate;

FIG. 4 is a graph showing the temperature dependence of the emission at 614 nm of a EuTTA/PMMA film;

FIG. 5 is a graph showing the variation of fluorescence signal with varying temperature over time;

FIG. 6 is a graph showing the variation of refractive index with changing temperature for water;

FIG. 7 is a graph showing temperature dependence of the emission at 440 nm of a terthiophene/PMMA film;

FIG. 8 is a graph showing the change in fluorescence of EuTTA/PMMA film in coated microplate well containing KOH upon dilution with water versus time; and

FIG. 9 is a graph showing the change in fluorescence of EuTTA/PMMA film in coated microplate well containing KI upon dilution with water versus time

**DETAILED DESCRIPTION**

The invention relates to assay methods, devices and systems for monitoring biological or chemical interactions, by providing means to monitor the temperature in at least one location in an array of locations. Additionally, the invention is compatible with optical detection principles known in the art such as fluorescence intensity, fluorescence anisotropy, fluorescence energy transfer, time resolved fluorescence, luminescence and combinations thereof, which may be used in the determination of interaction characteristics that do not have an effect on temperature of a location. According to the present invention, the interactions between and among chemicals, cells and biomolecules, can be detected by monitoring the temperature of at least one, but preferably more than one location in real time. Such temperature monitoring provides the ability to detect and measure those interactions between biomolecules and a second compound associated with heat generation or consumption.

A preferred aspect of the invention utilizes a substrate having a fluorescent material associated with the substrate. As used herein, the term "substrate" refers to a sample holder or container suitable for use in the measurement of interaction between biomolecules in an array of locations. Such as substrate, can include, for example, a microfluidics chip, a microplate or a microarray chip suitable for use in high throughput screening of biomolecules. The fluorescent material has a fluorescence that changes with changing temperature.

The fluorescent material may be associated with a substrate in a variety of ways. According to one aspect of the invention, the fluorescent material may be mixed with a solvent and applied to the substrate by spraying, dipping, coating, brushing and other methods that can form a uniform and reproducible coating on a substrate, which can be made from a variety of materials. The fluorescent material may be part of a composite material for optimum heat capacity and thermal conductivity. Structured composites can also be used to induce anisotropy in thermal conductivity to improve the heat transfer between the sample and the temperature sensitive material.

Alternatively, the fluorescent material can be manufactured in the form of a sheet or a film, which can be placed in contact with the substrate. Another way of associating the fluorescent material may be by incorporating the material into the structure of the substrate. For example, the fluorescent material could be impregnated into the material used to manufacture the substrate. Such impregnation methods are known in the art of manufacturing substrates such as microplates made from polymeric materials. As one example, a dye compound including the fluorescent material could be dispersed in a matrix of the material used to make the substrate. The matrix could be an organic matrix such as a polymeric material or an inorganic matrix made of sol-gel materials. The fluorescent material would not necessarily have to be incorporated into the entire structure of the substrate, and preferably, only a portion of the substrate would have the fluorescent material incorporated therein. For example, if the substrate is a microplate, it may be desirable for only the bottom portion of the microplate to have the fluorescent material incorporated therein. Alternatively, a temperature sensitive film can be made by forming a thin film of a matrix material and a fluorescent die using techniques such as casting, rolling, extruding and the like. The particular means of associating the fluorescent material with the substrate will depend at least upon the type of substrate desired and the type of material used to manufacture the substrate. Such substrate materials include, but are not limited to glass, quartz, silica, ceramics, metals, polymeric materials, and combinations thereof.

According to the present invention, the fluorescent properties of the fluorescent material associated with the substrate depend on temperature. Temperature dependent properties of the fluorescent coating or film can include the emission intensity at a given wavelength or over a range of wavelengths or the spectral characteristics of the emitted light.

Fluorescent dye compounds exhibiting high temperature dependence of their fluorescent properties are known in the art. For example, Europium (III) Thenoyltrifluoroacetonate trihydrate (EuTTA) shows a decrease of fluorescence intensity with increasing temperature. Alternatively, some compounds exhibit the reverse effect with increasing fluorescence intensity when temperature increases. The fluorescent layer or film can be made essentially transparent except for the portion of the spectrum where the fluorescent material is light absorbing. It will be understood, that a variety of fluorescent materials may be used in accordance with the present invention. EuTTA is a particularly preferred material. Other preferred materials include Rhodamine B, erythrosine B or terthiophene. Examples of other candidate materials that may be used in accordance with the invention include, but are not limited to, EuFOD, EuTFC, TbFOD, EuBA, EuTHD, EuHFC, EuDBM, EuTA, EuTFA, EuDCM, TTED, TbTTA, TbTFA, TbBA, TbTHD, TbAA, and combinations thereof.

The thickness and the concentration of luminescent dye of the temperature sensitive coating or film have an influence on the temperature sensitivity of the coating or film. In order to optimally detect the heat generation or consumption produced by the reaction in a sample, the heat capacity and thermal conductivity of the materials (coating and substrate or film) should be optimized. Therefore, the overall thickness of the temperature sensitive part of the substrate, such as a microplate or microarray chip, in thermal communication with the sample (e.g., bottom of the well + coating or film) are preferably thin (on the order of a fifty micrometers or less). For a given coating or film thickness, the concentration of the dye is preferably adjusted at the upper limit where absorption versus concentration relationship starts to depart from linearity.

Preferably, according to the present invention, microplates having thin portions at the bottom of each well are used. The thin bottom portion of a substrate can include, but is not limited to transparent polymers, polystyrene, polypropylene, UV transparent film, glass, metal and combinations thereof. It may be desirable to include a metal film in the bottom portion of the substrate such as aluminum foil because metal films have high thermal conductivity.

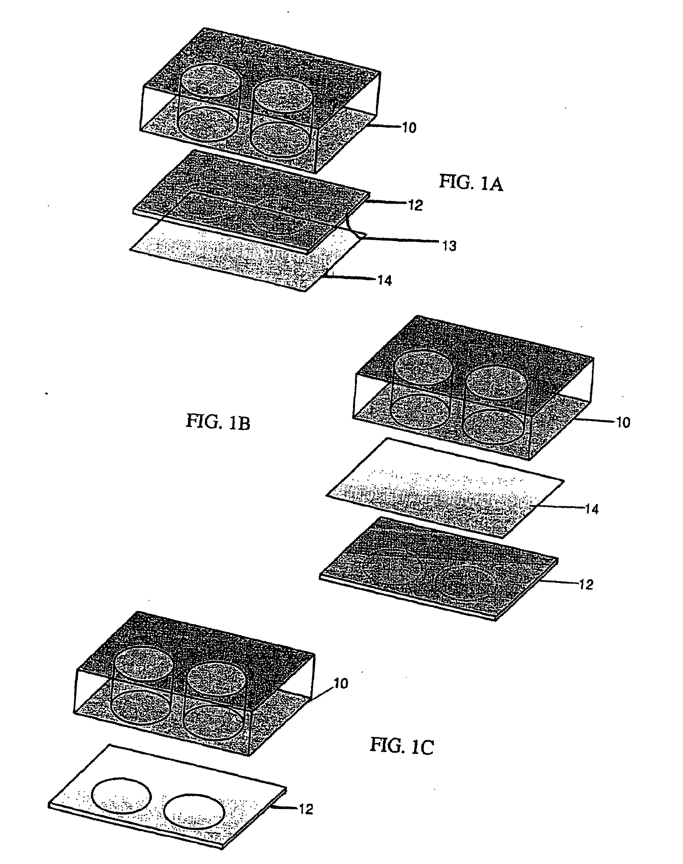

It will be appreciated that the fluorescent material can be positioned in a variety of locations with respect to a substrate. For example, as shown in FIG. 1A, a microplate 10 is shown having a bottom portion 12 having a bottom surface 13. A fluorescent film 14 is positioned on the bottom surface of the bottom portion of the microplate. In an alternative embodiment shown in FIG. 1B, the microplate 10 having a bottom portion 12 includes a fluorescent film 14 located between microplate wells and the bottom portion 12. In still another embodiment microplate 10 can include a bottom portion 12 incorporating the fluorescent material. As discussed above, such incorporation can involve impregnating the material used to make the bottom portion of the microplate, or alternatively, the bottom portion could be doped or coated with the fluorescent material.

In embodiments in which the substrate is a microplate, the fluorescent material can form continuous layer over the entire bottom portion of the substrate used to close wells or the fluorescent material may be associated with individual wells by coating or doping the individual wells with the fluorescent material.

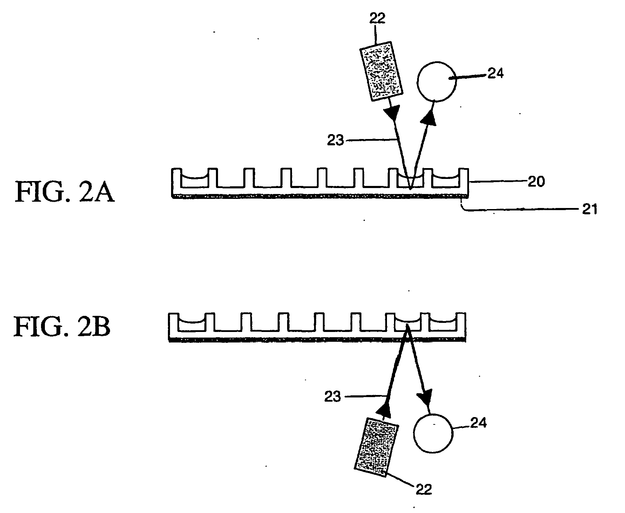

A wide variety of fluorometers and luminometers are used in biological assays or high-throughput screening of drug compounds by end-point or real-time reading of microtiter plates. FIGS. 2A and 2B show schematics of an exemplary temperature monitoring system according to the invention. FIG. 2A illustrates a setup including a microplate 20 having a fluorescent coating 21 utilizing a light source 22 that produces a light beam 23 directed at the fluorescent material 21 associated with the microplate 20. A detector 24 detects the light emitted by the fluorescent material associated with the microplate 20. A processor associated with a central processing unit (not shown) correlates the fluorescence with a temperature reading and provides a temperature indication in the wells of the microplate. FIG. 2A shows a setup in which the light source and the detector are positioned above the microplate wells. FIG. 2B shows a setup in which the light source 22 and the detector 24 are positioned below the microplate wells. It will be understood that the invention is not limited to a particular detector and light source configuration, and other configurations are within the scope of the invention. Additionally, while FIGS. 2A and 2B show a microplate, it will be understood that any suitable substrate for chemical or biological analysis can be utilized and incorporate a fluorescent material according to the present invention. Accordingly, the substrate could be a cuvette, a microarray, a microfluidics device or any other suitable substrate for processing chemical and biological materials.

According to the present invention, a substrate having a fluorescent material is used in such instruments by interposing a portion of the substrate incorporating the fluorescent material with an excitation beam and by detecting the emitted light at a particular wavelength or over a range of wavelengths. The emitted light property, for example, intensity at peak emission wavelength, provides a direct measure of the temperature of the coating or film at the bottom location being measured. This temperature can then be correlated to determine whether a biomolecule has reacted with a compound to create a positive or negative heat of reaction.

The invention is particularly useful for performing cell-based assays for detecting and monitoring metabolic changes induced by chemical or biochemical stimuli in cells. For example, if a microplate having a bottom portion including a fluorescent layer is utilized in accordance with the present invention, cells may be dispensed in each well with a volume of culture or nutritive medium. After the measurement instrument is thermally equilibrated, the temperature in at least one microplate well is real-time monitored by measuring the optical response of the temperature sensitive film or coating. By dispensing and mixing different compounds in each well and monitoring the temperature, it is possible to identify compounds that have an effect on thermogenic processes of the cells by impacting their metabolism. This protocol can be used, for example, to identify agonists and antagonists of therapeutically important membrane receptors such as G-protein coupled receptors, tyrosine kinase receptors, or nuclear receptors, inhibitors of enzymatic reactions and the like.

It is also within the scope of the invention to utilize a high density microarray chips for high throughput screening of biomolecules using an imaging system. As used herein, the term biomolecule includes a variety of biological materials, including, but not limited to amino acids such as DNA and RNA, peptides, proteins, oligonucleotides, lipids, or portions of cells. As noted above, because a typical target for drug action is with and within the cells of the body, cells themselves can provide a useful screening tool in drug discovery when combined with sensitive detection reagents. It thus would be useful to have a high throughput, high content screening device to provide high content spatial information at the cellular and subcellular level as well as temporal information about changes in physiological, biochemical and molecular activities. For example, if the chip substrate having a portion including a fluorescent layer or film is utilized in accordance with the present invention, cells may be dispersed and attached to the substrate in a high spatial density array. The optical response of the temperature sensitive coating or film can be measured using an imaging fluorometer including a charge coupled device, allowing multiplexed detection of the thermogenic effects of multiple compounds on identical cells or biomolecules. Alternatively, multiplexed detection of the thermogenic effects of the same compounds on different cells or biomolecules can be performed.

Further modifications of the invention could include combining temperature monitoring with another measurement such as time resolved fluorescence to provide additional characterization of the biological system preferably in the same instrument used to monitor the temperature.

Another aspect of the invention relates to compensating for temperature variations by using a fluorescent material having temperature dependent optical properties. Preferably, the fluorescent coating having temperature dependent optical properties is positioned proximate to the sensing area and is used to monitor refractive index changes associated with the biological or chemical samples. The temperature compensation system and method involves the measurement of the luminescent property of the coating that in turn gives a measure of the local temperature of the sensing area where the refractive index is being measured. Refractive index variations of the sensing area, which may include the substrate and/or the fluid containing the biological or chemical sample, can be calculated and compensated for in real time by adjusting for the local temperature contribution to the refractive index variations of the sensing area. In other words, if the refractive index variation over a temperature range of the substrate and the fluid are known, the variation in refractive index can be compensated for by adjusting the optical signal obtained for these variations.

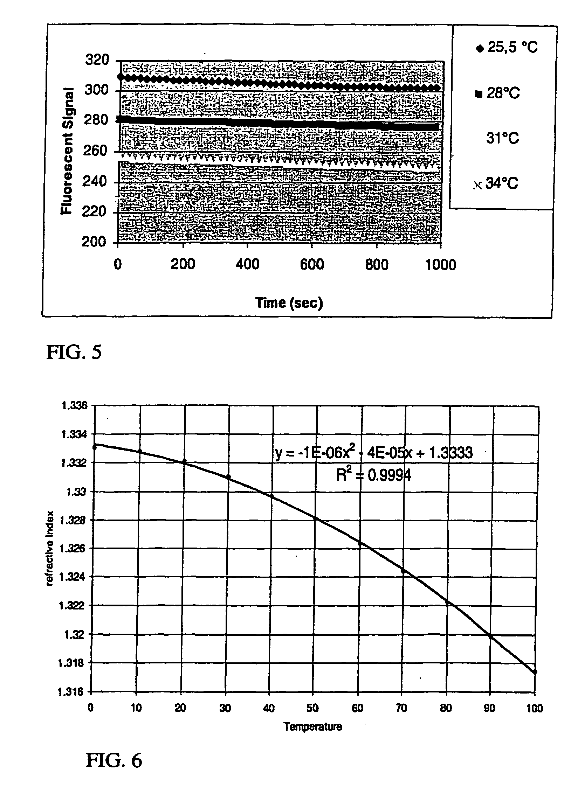

For example, the temperature dependence of the refractive index of water, which is routinely used for chemical and biological processing, is illustrated in FIG. 6. The variation of the refractive index of water with temperature is thus described by the following:

$$\Delta n / \Delta T = -(4 \times 10^{-5}) - (2 \times 10^{-6})T \qquad (1)$$

$$\text{at about } 20\ °C,\ \Delta n / \Delta T = -8 \times 10^{-5}\ \text{degree}^{-1} \qquad (2)$$

$$\text{at about } 40\ °C,\ \Delta n / \Delta T = -1.2 \times 10^{-4}\ \text{degree}^{-1} \qquad (3)$$

where n is the refractive index and T is the temperature. The system and the method of the present invention may be capable of compensating for variations of refractive index produced by temperature variations in an aqueous medium as low as $2.4 \times 10^{-6}$ at 20 °C (equation 2) and $3.6 \times 10^{-6}$ at 40 °C (equation 3).

The luminescent material which may be in the form of a layer on a surface of a substrate or embedded in the material forming the substrate according to the invention can be used advantageously in microplates, microarray chips, microfluidic devices and microbioanalytical devices where temperature of fluids is monitored during device operation or for detection purposes. For example, the temperature of nucleic acid samples undergoing PCR reaction or hybridization could be monitored in real time.

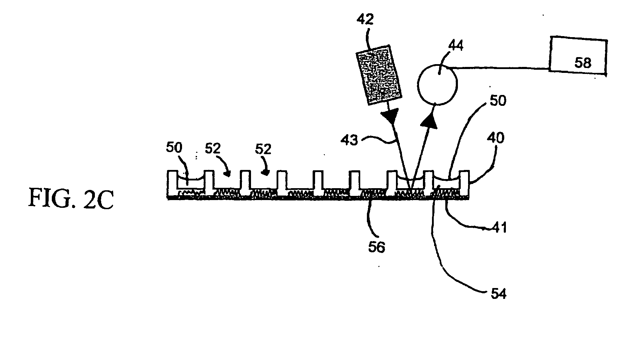

An example of an embodiment in which temperature and refractive index are monitored in real time is shown in FIG. 2C. As shown in FIG. 2C, a substrate 40, which in the embodiment shown is a microplate containing a number of wells 52, is in contact with a fluid 50 containing biomolecules, chemical or cells. The substrate 40 includes temperature dependent fluorescent material 41 in association with the substrate. As in the earlier described embodiments shown in FIGS. 2A and 2B, the fluorescent material 41 may be in the form of a film or layer, or it may be embedded into the material that forms the substrate 40. The substrate may include a sensing area 54, which in the embodiment shown in FIG. 2C is in the wells. It will be appreciated that the sensing area 54 is the area proximate to where the chemical reaction, biological reaction or metabolic change of a cell occurs. According to this aspect of the invention, a waveguide 56, which may be in the form of an optical fiber, a planar waveguide, a waveguiding film, a grating, a mirror, an interferometer, or other appropriate waveguiding structure is in association with the substrate. In the embodiment shown in FIG. 2C, the waveguide 56 is a grating. The waveguide may also include a combination of waveguiding structures. For example, the waveguide may include a waveguiding film and a grating in combination as disclosed in United States Patent Number 5,738,825. As shown in United States Patent Number 5,738,825, a waveguiding film and a separate diffraction grating are associated with each individual well in a microplate. Appropriate waveguiding films include, but are not limited to metal oxide materials such as silica, titania, titania-silica, alumina, and other suitable waveguiding materials.

Still referring to FIG. 2C, a light source 42 directs a light beam 43 toward the sensing area 54. Waveguide 56 directs the light beam towards detector 44 generated proximate the sensing area 54. A processor 58 in communication with the detector is associated with a central processing unit (not shown). The detector 44 will read the fluorescence generated by the fluorescent material proximate the sensing area 54, and the processor 58 determines temperature changes based on the fluorescence signal, which may, for example, be an increase or decrease in fluorescent intensity.

According to another aspect of the invention, the detector 44, or a separate detector (not shown), may measure the refractive index of the sensing area, which may include the fluid and the substrate surface. The processor 58 (or a separate processor) is operative to adjust the refractive index reading based on the change in temperature in the sensing area. For example, if the refractive index of the substrate material and the fluid are known over a temperature range, this information can be used to compensate the change in refractive index for the change in temperature and provide an compensated or corrected reading of the refractive index proximate the sensing area.

In devices utilizing Mach-Zehnder devices or photon sieves in contact or filled with a fluid, the temperature effect on refractive index of the fluid can be corrected by utilizing the fluorescence reading. Another important aspect is the time dependence of the temperature drifts that are likely to be different from the kinetics of the events to be measured. The fluorescent materials of the present invention can be used to provide a real time correction of the temperature drifts in the fluid and compensate for the difference between the kinetics of the events being measured.

In another aspect of the invention, a capillary or film containing at least one capillary can include the temperature dependent fluorescent materials of the present invention. For example, a thin coating (e.g., 20 microns or less) of a porous structure including the temperature sensitive coating can be provided. Alternatively, a porous membrane made from a suitable material, such as, for example, PMMA, can be filled with a temperature dependent fluorescent material. Either one of these structures can be utilized as a membrane for cell cultures and detection of a metabolic change in a cell such as a metastatic invasion, as the cell migrates through the capillary and the temperature of the capillary adjacent during migration is monitored.

The process steps of the invention described above can be carried out using conventional equipment known in the art, e.g., equipment commonly found in a bioanalytical laboratory. For example, the principle of the present invention can be utilized with conventional analysis equipment to detect temperature changes associated with a change in fluorescence. Without intending to limit the invention in any manner, the present invention will be more fully described by the following examples.

## EXAMPLES

### Example 1

EuTTA/PMMA Coating in Microplate Wells

EuTTA and PMMA (polymethylmethacrylate) were dissolved in a highly volatile solvent. For example, 2% w/w EuTTA, 2% w/w PMMA, and 96% w/w Methyl ethyl ketone (MEK) were mixed in a container. A 96 well microplate having a transparent polypropylene bottom (Coming, Inc. (catalog #9520), Coming, New York) was obtained, and an appropriate volume of the solution was deposited with a micropipette at the center of each plate well. To coat the bottom of a polypropylene plate with EuTTA/PMMA/MEK, approximately 15 $\mu$l of solution was used. A film was formed by solvent evaporation at room temperature and pressure. The coated plate was UV cured (365nm, 1J/cm$^2$) to stabilize the coating.

### Example 2

Absorption Spectrum of EuTTA/PMMA Coating

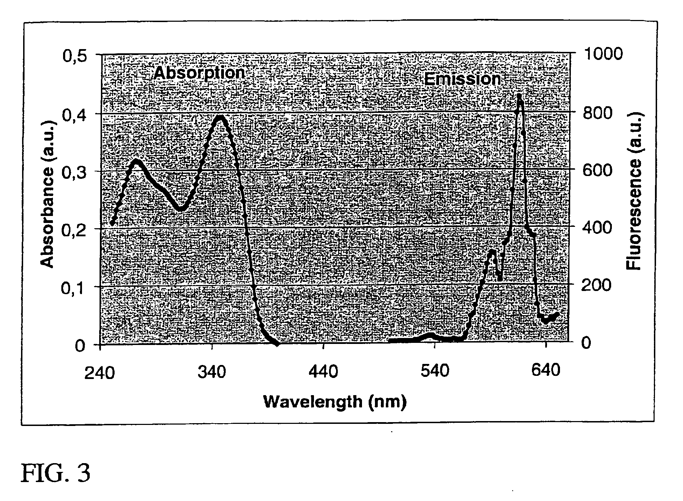

The absorption spectrum of the coating deposited on the microplate wells in Example 1 was measured every 2 nm with a SpectraMax® Plus (Molecular Devices Corporation, Sunnyvale, California) UV/VIS microplate spectrophotometer. The absorption spectrum extended from 200 to 400 nm and showed a maximum at 346 nm. The emission spectrum ranged from 500 to 650 nm and showed a maximum at 614 nm. The absorption and emission spectra are shown in FIG. 3.

### Example 3

Temperature Dependence of Fluorescence Signal from EuTTA/PMMA Coating

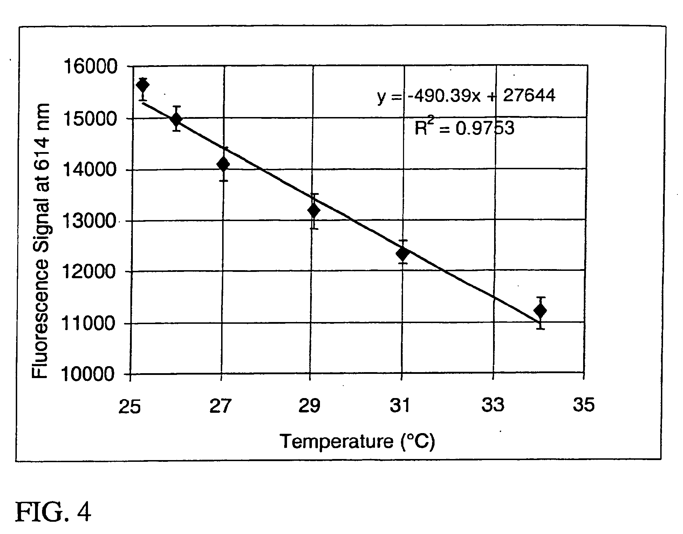

The fluorescence signal emitted by the coating deposited in Example 1 was measured at different temperatures. The microplate was heated and the fluorescence was measured at an emission wavelength of 614 nm using a SpectraMax® Gemini (Molecular Devices Corporation) dual-scanning microplate spectrofluorometer at an excitation wavelength of 355 nm over a temperature range from about 25 °C and 34 °C. The results in FIG. 4 show an approximate 3% decrease per °C for a 2% EuTTA/2% PMMA/96% MEK coating initial composition.

### Example 4

Evaluation of the Limit of Detection

To evaluate the limit of detection, a noise measurement was done using a bottom read set-up as shown in FIG. 2B using a Fluoroskan Ascent available from Labsystems. A microplate coated with EuTTA/PMMA was prepared in accordance with Example 1. One column (8 wells) of the microplate was filled with water and a kinetic measurement was performed at temperatures ranging from 25 °C to 35 °C. For each temperature point, 50 fluorescence measurements were performed at 20 seconds intervals. A graph of the results is shown in FIG. 5. Signal drift and signal noise was calculated by assimilating signal drift to a straight line and calculating its slope. The slope was equal to -0.0073/°C. The fluorescent signal measured was corrected according to the following formula (corresponding to the slope observed on the curves):

$$\text{Signal}_{corrected} = \text{Signal}_{raw} + 0.0073 \text{ X Temperature}$$

The standard deviation of 50 data points was calculated to be 0.285. Knowing that the fluorescent signal varies 9.4241 units per degree centigrade, the noise and also the method sensitivity was calculated to be 0.285/9.4241 or equal to 30 X 10$^{-3}$ °C.

### Example 5

Terthiophene/PMMA Coating in Microplate Wells

Terthiophene and PMMA (polymethylmethacrylate) were dissolved in a highly volatile solvent. For example, 2% w/w terthiophene, 2% w/w PMMA, and 96% w/w Methyl ethyl ketone (MEK) were mixed in a container. A 96 well microplate having a transparent polypropylene bottom (Coming, Inc. (catalog #9520), Coming, New York) was obtained, and an appropriate volume of the solution was deposited with a micropipette at the center of each plate well. To coat the bottom of a polypropylene plate with EuTTA/PMMA/MEK, approximately 15 μl of solution was used. A film was formed by solvent evaporation at room temperature and pressure. The coated plate was UV cured (365nm, 1J/cm$^2$) to stabilize the coating.

Example 6

Temperature Dependence of Fluorescence Signal from Terthiophene/PMMA Coating

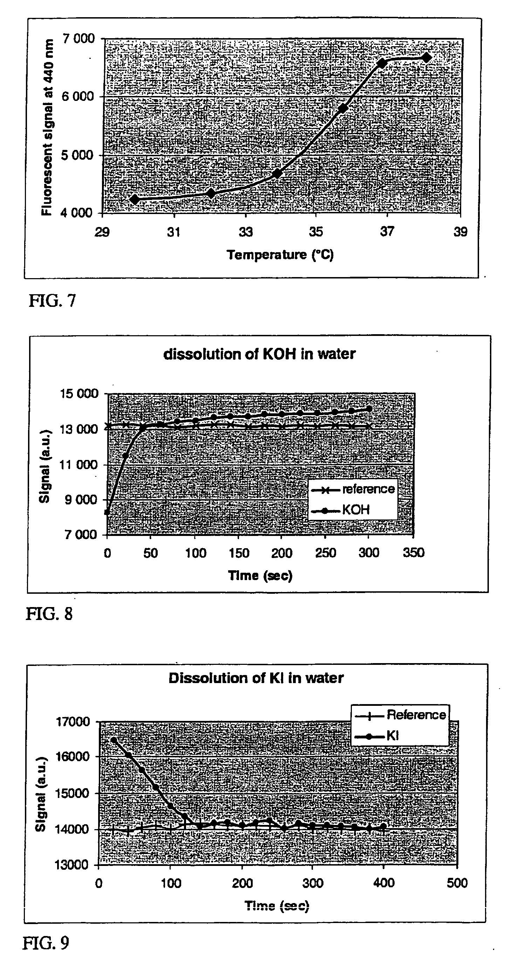

The fluorescent signal emitted by the coating deposited in Example 5 was measured at an emission wavelength of 440 nm with an excitation wavelength of 355 nm over a temperature range from 30 °C to 38 °C. The results are shown in FIG. 7 and show an approximate 10% fluorescence signal increase per degree centigrade between about 34 °C and 37 °C.

Others dyes such as Rhodamine B and Erythrosin B were also used and showed that a change in fluorescence associated with a temperature change in a reaction chamber could be monitored.

Example 7

Detection of Chemical Reactions

To demonstrate the principle that the change in fluorescence of a fluorescent material could be used to detect a chemical reaction between two compounds, an experiment was performed with chemicals having known endothermic or exothermic dissolution behavior in water.

First, eight wells of a 96 well microplate available from Coming, Inc., Coming, NY were coated with a solution of a EuTTA/PMMA in accordance with Example 1 above. The coated wells of the microplate were filled with 200 $\mu$l of water. KOH was added to seven of the eight coated wells, and one well was used as a reference well. The fluorescence signal from the wells was measured at an excitation wavelength of 355 nm and an emission wavelength of 614 nm over a 15 minute interval. FIG. 6 shows the temperature increase, represented by the change in fluorescence signal, due to the exothermic dissolution of KOH in water versus the reference well. A kinetic measurement monitoring the corresponding wells fluorescence signal with time was started immediately after the chemical addition at 25 °C. As expected, in case of an exothermic phenomenon, the signal decreases immediately and then goes back to its initial value, proving that the temperature increases due to the chemical addition and decreases progressively down to its initial value after the dissolution is complete.

Similarly, with an endothermic phenomenon as shown in FIG. 9, the fluorescence signal was increasing due to the chemical addition and went back progressively to its initial value. For example, a 15% increase of signal was observed during the dissolution of 30 mg iodide potassium (KI) in 200$\mu$l water. This example shows that it is possible to monitor and detect a chemical reaction between two compounds.

Example 8

Detection of Metabolic Response in a Biomolecule

To make in vitro cell culture, animal serum is usually added to the basic medium to provide to the cells nutriments and growth factor. A deprivation of this kind of serum leads to a slowing down of the cell metabolism. If serum is added after a deprivation period, the cell metabolism is reactivated and heat is generated.

The present experiment detects the response of CEM cells to serum addition in their culture medium after a serum deprivation period.

The culture medium is prepared as follows (each component is provided by GibcoBRL Life Technologie):

RPMI: 89.8%
Veal Fetal Serum: 9%
Antimycotic Antibiotic: 0.9%
HEPES: 0.1%
Sodium Pyruvate: 0.1%
Glucose: 0.1%

CEM cells are cultivated in the medium described above at 37 °C in an incubator with 5% of $CO_2$ atmosphere. Three to four hour before the beginning of the experiment, they are centrifuged and the medium is eliminated and replaced by a fresh one that does not contain any Veal Fetal Serum. The sample is divided in two parts of same volume: to the first one, veal fetal serum is added (these cells are not serum deprived) to reach 9% of serum in the total medium composition while to the second one, the same volume of medium (without veal fetal serum) is added. The two samples are put in the incubator again 3 to 4 hours. After this period of time, they are centrifuged, their media are eliminated and replaced by fresh media (with serum in the first case and without serum in the second case). They are counted and dispenses in the wells of a 384 well-microtiterplate (provided by Coming, Inc, reference 3712). The total dispensed volume per well is 50 $\mu$l and the cells number per well is $10^6$. Some additional wells are filled with the culture medium only (some wells with the medium containing serum and some others with the medium without serum). The external side of the microtiterplate bottom was previously treated with a EuTTA/PMMA coating using the same composition and deposition method than in Example 1.

The microtiterplate is installed in a bottom read fluorometer (Fluoroskan Ascent) temperature regulated at 37 C. The fluorescence signal from the wells is measured with the fluorometer for 190 seconds (one measurement every 10 seconds). Next, 40 $\mu$l of veal fetal serum at 37 °C is dispensed in every well, and the fluorescence signal from the wells is measured again (1 measurement every 10 seconds).

The fluorescence of the wells containing the serum deprived cells is expected to change after the serum injection while the fluorescence of the other wells (non deprived cells, medium with serum and medium without of serum) is not supposed to change, these later wells being the controls.

As can be seen in Table 1, the kinetic analysis revealed that 10 minutes after the serum injection, a 1.1% fluorescence change is observed from the wells containing the serum deprived cells, meaning that a heat is generated in these wells and detected. On the other hand, at the same time, the fluorescence signal from the wells containing the control solutions have a value similar to this before serum injection, meaning that the addition of serum does not generate any heat in these wells. After this 10 minutes, fluorescence from the wells containing the deprived cells tends to slowly increase becoming closer and closer to its initial value (before serum injection). This is in accordance with a stabilization of the cells after they get enough serum to reactivate their metabolism.

This result validates the use of temperature-dependent fluorescent coating to monitor cell metabolism activation.

Table 1

|  | Fluorescence signal before serum injection (au.) | Fluorescence signal 10 minutes after the serum injection (au.) |
|---|---|---|
| Medium with serum | 1178±3 | 1178±2 |
| Medium without serum | 1178±3 | 1178*3 |
| Non deprived cells | 1178±2 | 1178±3 |
| Serum deprived cells | 1176±2 | 1163±3 |

It will be apparent to those skilled in the art that various modifications and variations can be made to the present invention, For example, a variety of fluorescent materials and combinations of fluorescent materials that exhibit a changing fluorescence with changing temperature may be used in accordance with the present invention. For example, in addition to the fluorescent materials discussed in the specification, other fluorescent materials including, without limitation, Rhodamine B, Erythrosin B, and terthiophene can be used in accordance with the present invention.

**Claims**

1. A method for assaying or screening substances proximate a sensing area of a surface comprising:

   detecting a light signal generated proximate the sensing area;
   measuring the temperature proximate the sensing area;
   measuring the refractive index of the sensing area; and
   determining the change in refractive index of the sensing area due to the change in temperature and adjusting the light signal in accordance with the change in refractive index.

2. The method of claim 1, wherein the sensing area includes a substrate and a fluid in contact with the substrate.

3. The method of claim 2, wherein the substrate is a microplate, a microarray chip or a microfluidic chip.

4. The method of claim 2 or 3, wherein the fluid contains a cell or a biomolecule.

5. The method of any of claims 1 to 4, wherein a fluorescent material having a temperature-dependent fluorescence is proximate the sensing area.

6. The method of claim 5, wherein the fluorescent material is selected from the group consisting of EuTTA, Rhodamine B, Erythrosin B, terthiophene and combinations thereof.

7. The method of claim 5 or 6, wherein the fluorescent material is embedded in the substrate.

8. The method of claim 5 or 6, wherein the fluorescent material is in the form of a layer on a surface of the substrate.

9. The method of any of claims 5 to 8, wherein the substrate includes at least one capillary, and the fluorescent material is adjacent the capillary.


**Patentansprüche**

1. Verfahren zum Untersuchen oder Screenen von Substanzen in der Nähe eines Messbereichs einer Oberfläche, umfassend:

   Detektieren eines Lichtsignals, das in der Nähe des Messbereichs erzeugt wird;
   Messen der Temperatur in der Nähe des Messbereichs;
   Messen des Brechungsindex des Messbereichs; und
   Bestimmen der Änderung im Brechungsindex des Messbereichs aufgrund der Änderungen der Temperatur und Anpassen des Lichtsignals gemäß der Änderung des Brechungsindex.

2. Verfahren gemäß Anspruch 1, wobei der Messbereich ein Substrat und ein Fluid in Kontakt mit dem Substrat einschließt.

3. Verfahren gemäß Anspruch 2, wobei das Substrat ein Mikroplatte, ein Mikroarraychip oder ein Mikrofluidchip ist.

4. Verfahren gemäß Anspruch 2 oder 3, wobei das Fluid eine Zelle oder ein Biomolekül enthält.

5. Verfahren gemäß einem der Ansprüche 1 - 4, wobei ein fluoreszierendes Material mit einer temperaturabhängigen Fluoreszenz in der Nähe des Messbereichs ist.

6. Verfahren gemäß Anspruch 5, wobei das fluoreszierende Material ausgewählt ist aus der Gruppe, bestehend aus EuTTA, Rhodamin B, Erythrosin B, Terthiophen und deren Kombinationen.

7. Verfahren gemäß Anspruch 5 oder 6, wobei das fluoreszierende Material in das Substrat eingebettet ist.

8. Verfahren gemäß Anspruch 5 oder 6, wobei das fluoreszierende Material in Form einer Schicht auf einer Oberfläche des Substrates vorliegt.

9. Verfahren gemäß einem der Ansprüche 5 - 8, wobei das Substrat mindestens eine Kapillare einschließt und das fluoreszierende Material an die Kapillare angrenzt.


**Revendications**

1. Procédé de dosage ou de criblage de substances proches d'une zone de détection d'une surface comprenant :

   la détection d'un signal lumineux généré à proximité de la zone de détection ;
   la mesure de la température à proximité de la zone de détection ;
   la mesure de l'indice de réfraction de la zone de détection ; et
   la détermination du changement d'indice de réfraction de la zone de détection dû au changement de température, et l'ajustement du signal lumineux selon le changement d'indice de réfraction.

2. Procédé selon la revendication 1, dans lequel la zone de détection comprend un substrat et un fluide en contact avec le substrat.

3. Procédé selon la revendication 2, dans lequel le substrat est une microplaque, une puce à micro-réseau ou une puce microfluidique.

4. Procédé selon la revendication 2 ou 3, dans lequel le fluide contient une cellule ou une biomolécule.

5. Procédé selon l'une quelconque des revendications 1 à 4, dans lequel un matériau fluorescent ayant une fluorescence dépendant de la température est proche de la zone de détection.

6. Procédé selon la revendication 5, dans lequel le matériau fluorescent est choisi parmi le groupe consistant en EuTTA, Rhodamine B, érythrosine B, terthiophène et des combinaisons de ceux-ci.

7. Procédé selon la revendication 5 ou 6, dans lequel le matériau fluorescent est intégré dans le substrat.

8. Procédé selon la revendication 5 ou 6, dans lequel le matériau fluorescent est sous la forme d'une couche sur une surface du substrat.

9. Procédé selon l'une quelconque des revendications 5 à 8, dans lequel le substrat comprend au moins un capillaire, et le matériau fluorescent est adjacent au capillaire.

FIG. 1A

—10

—12

—13

—14

FIG. 1B

—10

—14

—12

—10

FIG. 1C

—12

FIG. 2A

FIG. 2B

FIG. 2C

FIG. 3

FIG. 4

FIG. 5

FIG. 6

FIG. 7

FIG. 8

FIG. 9